# EUROPEAN PATENT APPLICATION

(11) **EP 2 368 428 A1**
(43) Date of publication of application: **28.09.2011**
(21) Application number: 10305261.9
(22) Date of filing: 16.03.2010
(51) Int. Cl.: A01K 67/027, C12Q 1/68, C12N 9/20

(54) **Obtention of a rex animal by molecular methods based on the alteration of the LIPH function**

(71) Applicant: INSTITUT NATIONAL DE LA RECHERCHE AGRONOMIQUE, 75338 Paris (FR)
(72) Inventor: Cribiu, Edmond-Paul, 91380 Chilly Mazarin (FR); Allain, Daniel, 31320 Castanet Tolosan (FR); Deretz, Séverine, 79270 Frontenay Rohan (FR); Auvinet, Gérard, 17700 Puyravault (FR); Diribarne, Mathieu, 92160 Antony (FR); Chantry-Darmon, Céline, 92160 Antony (FR); Rogel-Gaillard, Claire, 75013 Paris (FR); Mata, Xavier, 92160 Antony (FR); Guerin, Gérard, 78330 Fontenay le Fleury (FR)
(74) Representative: Cabinet Plasseraud

(57) **Abstract**

The present invention concerns *an in vitro* method for identifying a fur-bearing animal having essentially or exclusively down hair, wherein said method comprises the step of step of detecting the presence of a mutation in the lipase H (LIPH) gene in a biological sample obtained from said fur-bearing animal.

## Description

### Field of the invention

The invention relates to the identification and the obtaining of an animal having exclusively a coat with a rex phenotype. It covers the field of fur-bearing animals and, preferably, to a method for identifying an animal leading to a coat made of mainly or exclusively down hair. The invention includes the use of molecular methods based on (a) mutation(s) in a gene involved in a modification in the normal functioning of hair follicles resulting in disturbing the hair growth process. This alteration is considered as an advantage to the rabbit industry.

### Description of prior art

Contrarily to humans, most fur-bearing animal such as rabbits have three different types of hair in their coats related to specific natural functions. The longest type forms the outer fur called "guard hair" generated from "central primary hair follicles", a middle layer is named "awn hair" from "lateral primary hair follicles" and the shortest "down hair" from "secondary hair follicles", also known as undercoat with very thin shafts. Hair follicles are organized in groups made of a "trio group" composed of a central primary hair follicle and two lateral primaries with secondary hair follicles within the trio group.

The "rex" phenotype known since 1929 in the rabbit (*Oryctolagus cuniculus*), refers to animals having essentially down hair -i.e. secondary follicles hair-amazingly soft to touching. Since then, "rexoid" phenotypes have been also discovered in many other fur-bearing animal such as *Mus musculus, Rattus norvegicus, Felis catus, Mesocricetus auratus, Cavia porcellus.* Even if a number of mutations have been identified in different genes, the genetic basis of this phenotype is heterogeneous and still poorly understood.

In rabbits the rex phenotype of the coat is characterized by:
1) a notably reduced number of guard hair and an increased number of down hair which can lead to an extreme phenotype with a total absence of guard hair (a guard hair is defined as a fibre having a fibre diameter higher than 30µm and a length of a minimum of 30mm).
2) a large shortening of residual guard hair when present and a small shortening of down hair resulting by a light or a null difference in the length of both residual guard hair and downs, and
3) a curling of whiskers.
These three criterias are all used to assess and categorize rabbits having or not a rex coat phenotype. The quality of the fur of the rex coat phenotype confers to these animals an economic advantage and is selected by specialized breeders.

### Summary of the invention

The present invention relates to a method for identifying a fur-bearing animal having at least 95 % of down hair, wherein said method comprises the step (i) of detecting the presence of a mutation in the lipase H (LIPH) gene in a biological sample obtained from said fur-bearing animal.

In another particular embodiment, the method according to the present invention further comprises a step (i'), before step (i), of transformation of cells of said animal for presenting a mutation in the LIPH gene.

The present invention also relates to non-human transgenic fur-bearing animal obtained by the method according to the present invention and including step (i').

The present invention further relates to the use of a compound that inhibits specifically the expression of LIPH gene for obtaining fur-bearing animals having at least 95% of down hair.

### Brief Description of the Drawings

**Figure 1****:** Flanking sequences of the rex 1362de1A mutation in exon 9. By comparison with the "wild type" (non rex) genotype, a deletion of an adenine in both alleles (homozygous animals) of rex individuals has been observed. The heterozygous state shows an overlap of both rex and "wild type" alleles.
**Figure 2** **:** Construction of rabbit reference families according to a backcross model.
   "RR" et "Rr" respectively refer to Wild types and "r"refer to rex type.
**Figure 3** **:** Fine mapping of the rex trait (INRAR).

### Description of the Preferred embodiments

The inventors have established that a rabbit displaying a frameshift mutation in the exon 9 of the LIPH gene have a Rex phenotype coat with an increased proportion of down hair

The inventors have shown that the expression of a mutated LIPH protein results in a rex phenotype. Thus the invention provides a method for identifying a fur-bearing animal having at least 95 % of down hair, wherein said method comprises the step (i) of detecting the presence of a mutation in the lipase H (LIPH) gene in a biological sample obtained from said fur-bearing animal.

As used herein, the term "down hair" refers to a fibre having a diameter of less than 30 µm, preferably less than 20 µm and a length of a maximum of 25 mm, i.e. shorter than 25 mm, preferably shorter than 20 mm.

As used herein, the term "fur-bearing animal" refers to a mammal selected in the group comprising sheep, goat, mouflon, camel, alpaca and south American camelids (llama, guanaco, vicuna), cow, horse, beaver, fox, mink, muskrat, nutria, weasel, polecat and rabbit. Preferably, said fur-bearing animal is a rabbit and most preferably said fur-bearing animal is *Oryctolagus cuniculus.*

Preferably, said fur-bearing animal has at least 95% of down hair, more preferably at least 98% of down hair, even more preferably 100% of down hair.

Preferably, said fur-bearing animal has the rex phenotype.

As used herein, the expression "biological sample" refers to solid tissues such as, for example, a tissue biopsy; and to fluids or excretions such as for example, buccal swab, sputum, induced sputum, blood, serum, plasma, urine. The biological sample may be obtained from any cell source or tissue biopsy. Non-limiting examples of cell sources available include without limitation blood cells, buccal cells, epithelial cells, fibroblasts, or any cells present in a tissue obtained by biopsy. Cells may also be obtained from body fluids, such as blood, plasma, serum or lymph.

As used herein, the term "lipase H (LIPH)" corresponds to the enzyme catalyzing the production of 2-acyl lysophosphatidic acid (LPA), which is a lipid mediator with diverse biological properties that include platelet aggregation, smooth muscle contraction, and stimulation of cell proliferation and motility. Said LIPH gene can be simply identified by the skilled person according to its general knowledge. Typically, the LIPH gene can be detected by amplification and the LIPH protein can be detected with the mean of an antibody.
As an example, one can cite :
➢ the LIPH gene from *Mus musculus* (the cDNA encoding LIPH isoform 1 is identified as SEQ ID N°3, the cDNA encoding LIPH isoform 2 is identified as SEQ ID N°5, LIPH isoform 1 is identified as SEQ ID N°4, LIPH isoform 2 is identified as SEQ ID N°6), and
➢ the LIPH gene from *Oryctolagus cuniculus* (the cDNA encoding LIPH is identified as SEQ ID N°1 and the protein LIPH is identified as SEQ ID N°2).

DNA may be extracted using any methods known in the art, such as described in Sambrook et al., 1989. RNA may also be isolated, for instance from tissue biopsy, using standard methods well known to the one skilled in the art such as guanidium thiocyanate-phenol-chloroform extraction.

As used herein, the term "mutations" correspond to any modification in the sequence of the original nucleic acid sequence. These mutations comprise small-scale mutations, or large scale mutations. Small scale mutations are those affecting a gene in one or a few nucleotides, including point mutations, insertions or deletions of one or more extra nucleotides in the DNA. Point mutations can be missense and nonsense mutations. Large scale mutation in the genomic structure, such as gene duplications, deletions, or mutations whose effect is to juxtapose previously separate pieces of DNA, potentially bring together separate genes to form functionally distinct fusion genes. These last mutations include chromosomal translocations, interstitial deletions, chromosomal inversions and loss of heterezygosity.

Typically, the mutation in the LIPH gene may be detected by analyzing a LIPH nucleic acid molecule. In the context of the invention, LIPH nucleic acid molecules include mRNA, genomic DNA and cDNA derived from mRNA. DNA or RNA can be single stranded or double stranded. Preferably, LIPH nucleic acid is genomic DNA, or RNA. LIPH nucleic acid molecules may be utilized for detection by amplification and/or hybridization with a probe, for instance.

A LIPH gene mutation according to the invention may be selected in the group comprising insertions, deletions, and point mutations corresponding to missense mutations and nonsense mutations. Preferably the mutation of LIPH gene according to the invention is a non silent mutation. Preferably, the mutation of LIPH gene according to the invention is a deletion. More preferably, this mutation is a deletion on exon 9 the lipase H gene. Most preferably, this mutation is 1362de1A mutation in exon 9 of lipase H gene.

As used herein, the expression "mutation in the LIPH gene" corresponds to a modification of the wild-type LIPH gene sequence so that:
a) its expression leads to a corresponding truncated lipase H, or
b) its expression leads to a corresponding protein which has not the same function of the wild type lipase H, or
c) the LIPH gene is not expressed.

Typical techniques for detecting the presence of a mutation may include restriction fragment length polymorphism, hybridization techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridization, ligation chain reaction, mini-sequencing, DNA "chips", allele-specific oligonucleotide hybridization with single or dual-labelled probes merged with PCR or with molecular beacons, and others.

Advantageously, the mutation is detected on the cDNA of the LIPH gene by either PCR and sequencing or SNP-array, both being well known for the skilled person.

In molecular biology, a SNP array is a type of DNA microarray which is used to detect polymorphisms within a population. The basic principles of SNP array are the same as the DNA microarray. These are the convergence of DNA hybridization, fluorescence microscopy, and solid surface DNA capture. The three mandatory components of the SNP arrays are: i) the array that contains immobilized nucleic acid sequences or target; ii) one or more labelled Allele specific oligonucleotide (ASO) probes; and iii) a detection system that records and interprets the hybridization signal (see in Sheils, O., Finn, S. and O'Leary J. (2003) "Nucleic acid microarray: an overview", Current Diagnostic Pathology. 9:155-158).

As used herein, a "non silent mutation in the LIPH gene" corresponds to a mutation leading to polypeptide having a difference of at least one amino acid with the wild type LIPH polypeptide and which has a different function and/or conformation. Accordingly, said mutation in the LIPH gene may be detected at the protein level.

Said mutation may be detected according to any appropriate method known in the art. In particular a sample, such as a tissue biopsy, obtained from a subject may be contacted with antibodies specific of the mutated form of LIPH, i.e. antibodies that are capable of distinguishing between a mutated form of LIPH and the wild-type protein (or any other protein), to determine the presence or absence of a mutation in LIPH.

In a particular embodiment, the present invention relates to a method of identifying a fur-bearing animal having at least 95 % of down hair, wherein said method comprises the step (i) of detecting the presence of a mutation on each allele of the LIPH gene coding for the polypeptide having the sequence SEQ ID N°2 in a biological sample obtained from said fur-bearing animal.

The present invention also provides a method for identifying a fur-bearing animal having at least 95 % of down hair, wherein said method comprises the step of:.
(i) detecting the presence of a mutation in the lipase H (LIPH) gene in a biological sample from said fur-bearing animal, and/or
(ii) detecting the presence of a mutation in the LIPH protein in a biological sample from said fur-bearing animal.

Preferably, step (ii) is a step of detecting the presence of a truncated LIPH protein in a biological sample from said fur-bearing animal.

In another particular embodiment, the method according to the present invention further comprises a step (i'), before step (i), of transformation of cells of said animal for presenting a mutation in the LIPH gene or expressing a truncated LIPH protein. Preferably, said step (i') is a step of mutagenesis.

The present invention also relates to non-human transgenic fur-bearing animal obtained by the method according to the present invention and including step (i').

Preferably, said animal is a rabbit. More preferably, said animal is *Oryctolagus cuniculus.*

Preferably, detecting the presence of a mutation in the LIPH protein is made thanks to antibodies. Antibodies that specifically recognize a mutated LIPH are thus also part of the invention. The antibodies are specific of mutated LIPH, that is to say they do not cross-react with the wild-type LIPH. The antibodies of the present invention may be monoclonal or polyclonal antibodies, single chain or double chain, chimeric antibodies, humanized antibodies, or portions of an immunoglobulin molecule, including those portions known in the art as antigen binding fragments Fab, Fab', F(ab')2 and F(v). They can also be immunoconjugated, e.g. with a toxin, or labelled antibodies. The antibody can be labelled with a tag, so that the skilled man is capable of identifying a mutated LIPH. Implementing such identification falls within the ability of the skilled man. Whereas polyclonal antibodies may be used, monoclonal antibodies are preferred for they are more reproducible in the long run. Procedures for raising "polyclonal antibodies" are also well known. Polyclonal antibodies can be obtained from serum of an animal immunized against the appropriate antigen, which may be produced by genetic engineering for example according to standard methods well-known by one skilled in the art. Typically, such antibodies can be raised by administering mutated LIPH subcutaneously to New Zealand white rabbits which have first been bled to obtain pre-immune serum. The antigens can be injected at a total volume of 100 µl per site at six different sites. Each injected material may contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed by Harlow et al. (1988) which is hereby incorporated in the references.

A "monoclonal antibody" in its various grammatical forms refers to a population of antibody molecules that contains only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, e.g. a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al., 1988). Monoclonal antibodies (mAbs) may be prepared by immunizing purified mutated TXAS into a mammal, e.g. a mouse, rat, human and the like mammals. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody. This standard method of hybridoma culture is described in Kohler and Milstein (1975).

While mAbs can be produced by hybridoma culture the invention is not to be so limited. Also contemplated is the use of mAbs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No.; European Patent Publications No. 0239400 to Winter et al. and No. 0125023 to Cabilly et al.

Antibody generation techniques not involving immunisation are also contemplated such as for example using phage display technology to examine naive libraries (from non-immunised animals); see Barbas et al. (1992), and Waterhouse et al. (1993).

Antibodies raised against mutated LIPH may be cross reactive with wild-type LIPH. Accordingly a selection of antibodies specific for mutated LIPH is required. This may be achieved by depleting the pool of antibodies from those that are reactive with the wild-type LIPH, for instance by submitting the raised antibodies to an affinity chromatography against wild-type LIPH.

The present invention further relates to the use of a compound that inhibits the expression of LIPH gene for obtaining fur-bearing animals having at least 95% of down hair. Typically, said inhibitor of LIPH gene expression is an agent downregulating LIPH expression. Typically, agents downregulating LIPH gene expression comprise a nucleic acid which interferes with the expression of LIPH gene. Such compound may be an antisense molecule or a vector comprising said antisense molecules.

Antisense molecules are complementary strands of small segments of mRNA. Methods for designing effective antisense molecules being well known (see for example US6165990), it falls within the ability of the skilled artisan to design antisense molecules able to downregulate the expression of LIPH gene. Further examples are RNA interference (RNAi) molecules such as, for example, short interfering RNAs (siRNAs) and short harpin RNAs (shRNAs). RNAi refers to the introduction of homologous double stranded RNA to specifically target a gene's product, in the present case LIPH mRNA, resulting in a null or hypomorphic phenotype. Methods for designing effective RNAi molecules being well known (see for review Hannon and Rossi Nature. 2004 Sep 16; 431 (70006): 371-8) it falls within the ability of the skilled artisan to design RNAi molecules able to down-regulate LIPH gene expression.

In a further embodiment of the invention, the inhibitor of LIPH gene expression is an antibody against LIPH or a fragment or derivative thereof which inhibits LIPH biological activity. The person skilled in the art will be aware of standard methods for production of such specific antibody. For example, specific antibodies or biologically active fragments or derivatives thereof may be generated by immunizing an animal, for example KO LIPH mice, with LIPH or LIPH fragments and by selecting the antibodies which bind to LIPH. The person skilled in the art will be aware of standard methods for production of both polyclonal and monoclonal antibodies and biologically active fragments and derivatives thereof. By biologically active fragment or derivative thereof, it is meant able to bind to the same epitope as the antibody, in the present case an epitope of LIPH.. Antibody fragments, particularly Fab fragments and other fragments which retain epitope-binding capacity and specificity are also well known, as are chimeric antibodies, and "humanized" antibodies, in which structural (not determining specificity for antigen) regions of the antibody are replaced with analogous or similar regions from another species. Thus antibodies generated in mice can be "humanized" to reduce negative effects which may occur upon administration to human subjects. The present invention therefore comprehends use of antibody specific for LIPH which include F(ab')₂, F(ab)₂, Fab, Fv and Fd antibody fragments, chimeric antibodies in which one or more regions have been replaced by homologous human or non-human portions. The person skilled in the art will also be aware that biologically active antibody derivatives such as for example ScFv fragments and divalent ScFv-type molecules can be prepared using recombinant methods.

Preferably, said inhibitor of LIPH gene expression is an oligonucleotide, selected in the group comprising anti-sense DNA and RNA molecules, ribozymes, siRNA and aptamers.

More preferably, said inhibitor of gene LIPH gene expression is a siRNA with a sequence complementary to SEQ ID N° 1 or a fragment thereof. Any sequence with siRNA characteristics can be used to design proper sequences preferably in the 3'UTR region of the gene.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### Example

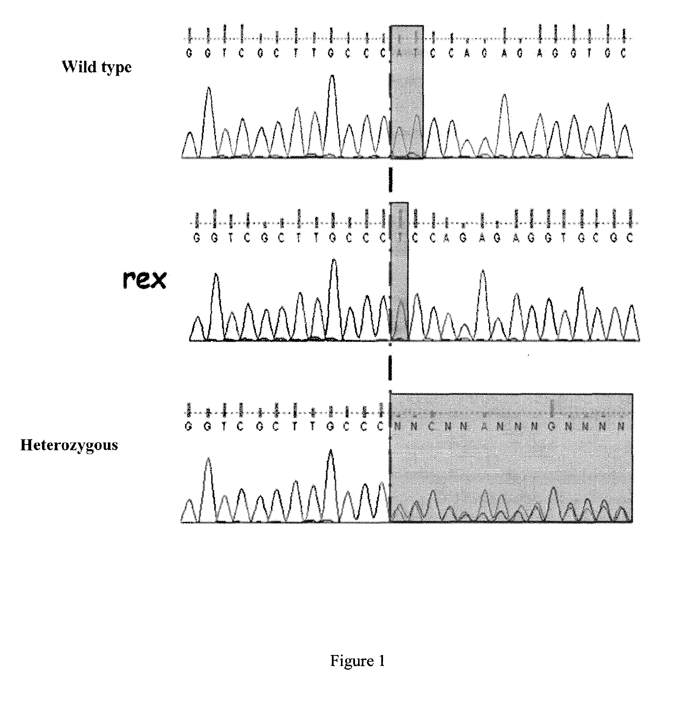

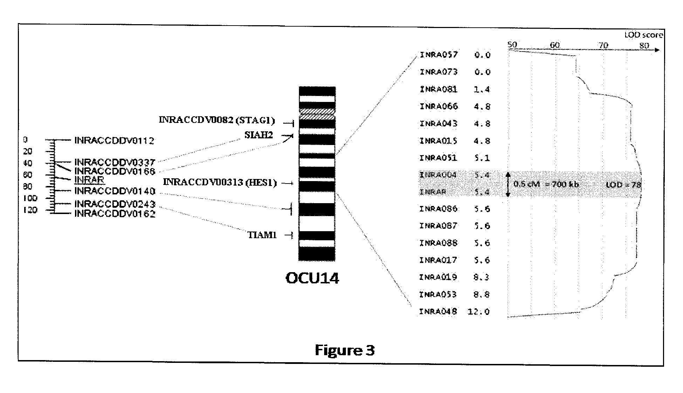

The Inventors have segregated rabbit lines with the rex phenotype. A positional cloning approach was chosen to map the rex trait. A first genome scan was carried out with a limited panel of microsatellites. The rex trait was thus mapped on the rabbit chromosome 14 with an interval of 40 centimorgan (cM). A further 172 microsatellites markers were identified in this region of which 68 were genotyped on the panel of rabbit families for fine mapping. The rex trait was finally localized within a 0.5cM interval with a LOD (logarithm of odds) score of 78 (Figure 3). In the last 700 kb interval, the LIPH gene was identified as a candidate gene due to the existence in the literature of a Knock Out mouse for this gene leading to a defect in the hair development. By sequencing the complete coding region of the LIPH gene in rex and non rex rabbits, a deletion of 1 nucleotide located in the exon 9 in a homozygous state in rex rabbits has been identified (Figure 1).

### 1. Rabbit families.

### a. Mating plan

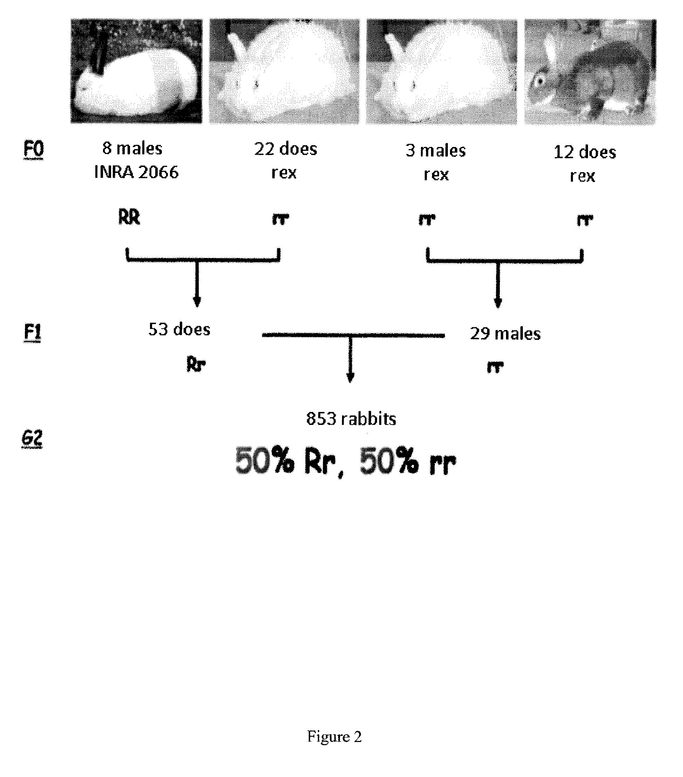

Three rabbit INRA strains were used to build three generation families based on a backcross model (figure 2). Two crosses were carried out to produce the F1 generation : i) eight INRA 2066 wild type (RR) males with 22 rex does (rr) and ii) three rex males (rr) with 12 rex does (rr). The 53 F1 does (Rr) from the i) cross were then mated with the 29 F1 males (rr) from the ii) cross, which produced 853 G2 rabbits with theoretically 50% of homozygous rex types (rr) and 50% of heterozygous showing wild phenotype (Rr).

### b. Phenotyping rabbits for the rex trait.

Rabbits were phenotyped for coat texture by analyzing a strand of hair. Measurements of maximum and minimum length coat and average diameter of the hair were performed. A subset of 99 G2 rabbits were further analysed (table 1) for mean fiber diameter and content of coarse fibres (having a diameter > 30µm) according OFDA methodology (Rafat et al, 2007).

**Table 1: OFDA measurements of mean fibre diameter and content of coarse fibres in normal and rex coat rabbits.**

| Rabbit coat type | N | Mean fibre diameter (µm) | Coefficient of variation (%) of fibre diameter | % coarse fibre (diameter>30µm) |
|---|---|---|---|---|
| Normal coat (Rr) | 54 | 15.2 ± 0.6 | 42.5 ± 6.4 | 2.3 ± 0.8 |
| Rex coat (rr) | 45 | 15.3 ± 0.5 | 32.1 ± 6.2 | 1.0 ± 0.5 |

### c. Genotyping

DNA was extracted from peripheral blood mononuclear cells recovered, using a protocol described by Jeanpierre (1987), from 8ml of blood sampled on ethylenediamine- tetraacetic acid (EDTA) (K3EDTA,Vacutainer B-D;Becton Dickinson, Rutherford, NJ, USA) by cardiac puncture. A low-cost technique described by Schuelke (2000) was applied for the genotyping of microsatellites. The polymerase chain reaction (PCR) reaction was carried out with three primers, including a primer specific for the locus to be amplified and extended by a universal sequence of 17 nucleotides (5'- GACCGGCAGCAAAATTG-3' identified as SEQ ID N°7), a reverse primer specific for the locus and a universal primer of 17 nucleotides that was 5'-labelled by 6-Fam, Hex or Tet (MWGAGBiotech, Ebersberg,Germany). 1. Results were analysed with the Genetic Profiler v1.1 software (ABIPrism377A sequencer; Applied Biosystems, FosterCity, CA, USA) or the Genotyper software (MegaBACE1000 sequencer; AmershamBiosciences, New Haven,CT,USA). Genotyping data were checked using an in-house program (A.Neau, personal communication), which analyzed the consistency of the allele distribution of each marker according to pedigrees.

### 2. Primo-localization of the rex gene

A genome scan was achieved by genotyping 187 G2 rabbits for the 98 microsatellites markers of the genetic rabbit map (Chantry et al, 2005).

The CRI-MAP 2.4 software (Green et al. 1990) was used to build the genetic map. The first step identified linked markers by a two-point analysis with the two-point option. In the second step, the linkage groups were examined by multipoint analyses using the build and flipsn options. Linkage data were merged with cytogenetic mapping data to confirm the order of markers or to identify linkage of isolated or weakly informative markers. These various analyses were performed with a LOD score of 3 (lowered to 1.8 in a few cases where the cytogenetic position of the markers made it possible to confirm the linkage). The size of the genetic map was calculated by adding up the genetic distances of all linkage groups plus 15 cM at both ends of each group and including 15 cM on both sides of each unlinked marker.

### 3. Fine mapping of the rex gene

### a. Characterization of microsatellite markers

Additional microsatellite markers localized in the primo localization interval were produced by screening BACs (large fragments of DNA) and *by in silico* approaches.

The BAC rabbit library known as the "LBAB" was built at the Laboratory of Radiobiology and Genome Study (LREG) at INRA in Jouy-en-Josas (Rogel-Gaillard et al., 2001). This library was constructed from genomic DNA extracted from lymphocytes of a male New Zealander rabbit and contains 84,480 clones with an average size insert of 110 kb (vector pBeloBAC11), which corresponds to 3 genome equivalents (3X). The genes of the human region corresponding to the interval of primary localization in the rabbit were listed by comparative mapping. Expressed sequence tag (EST) for these rabbits genes were retrieved from http://bioinfo.genopole-toulouse.prd.fr/iccare/. Primers for these ESTs were determined using the software Primer3 (Rozen and Skaletsky, 2000) and used to screen the rabbit BAC library LBAB. Screening of the BAC library was done in 2 steps, with an initial set of 47 PCR from superpools and a second set of 44 PCR DNA on 24 plates belonging to the superpool selected in step 1. PCR were performed in a final volume of 10 uL with 20-40 ng DNA pools, 2 mM MgCl2, 1X buffer, 0.25 mM dNTP, 1pmol of each primer and 1U of enzyme Taq polymerase Goflexi. The PCR conditions were as follows: 5 min at 95°C followed by 35 cycles of 30 sec at 95°C for denaturation, 30 seconds at 55°C for hybridization and 30 sec at 72° C for elongation, followed by a final elongation of 5 min at 72°C. The PCR products were analyzed by electrophoresis on agarose gel. The PCR conditions were then optimized by varying the temperature of hybridization and / or the number of cycles. The identity of the expected EST contained in each selected BAC was confirmed by sequencing. The subclones were amplified by PCR with its specific primers. Then, amplicons were purified using columns (Wizard SV Gel and PCR Clean-Up System, Promega). The sequencing reactions were performed using the Big Dye Terminator kit (Perkin Elmer) according to the following protocol. 1 to 3 ng of purified DNA are mixed with 10 pmol of the forward primer used to amplify the fragment of EST, and 4 uL of Big Dye Terminator mix in a final reaction volume of 12 uL. The DNA is then amplified by PCR. 1 min of denaturation at 94°C then 35 cycles comprising 10 seconds of denaturation at 94°C, 5 seconds at 50°C for hybridization and 4 min for primer elongation at 60°C. The chromatograms were read with the software BioEdit (http://www.mbio.ncsu.edu/BioEdit/bioedit.htmL) and compared to sequences present in databases using the BLASTN program (http://www.ncbi.nlm.nih.gov/BLAST/) (Altschul et al. 1990). For sequences of genes known to humans or other species, values lower than 10⁻⁵ for the parameter "e-value", were considered acceptable to confirm the identity of the rabbit BAC clones isolated.

DNA from BAC pools was digested with Sau3A. Digested DNA was run through a 1% low-melting agarose (ICN) gel and fragments ranging from 700 to 1200 base pairs (bp) were selected. Agarose gel containing the size-selected DNA fragments was cut and melted at 65°C for 15 min followed by incubation for 1 h at 42°C in the presence of 1.5 unit GELase (Epicentre)/100 mg agarose gel. DNA was extracted by phenol/chloroform, ethanol precipitated for 15 min at 80°C with 0.3 M sodium acetate (pH 5.2), and 20 µg glycogen as DNA carrier, and DNA pellets were rinsed with 70% ethanol. DNA concentration was estimated by migration electrophoresis through a 1% agarose gel with a quantitative DNA molecular weight marker (Marker XIV, Roche Diagnostics GmbH). Digested DNA fragments were ligated to the vector pGEM-4Z previously linearized by BamH1 and dephosphorylated. Ligations were performed at 8°C for 16 h in a total volume of 20 µl with 100 ng digested DNA, 100 ng vector, and 200 units of T4 DNA ligase with the associated 1 buffer (Biolabs). Ligation samples were incubated at 65°C for 10 min to inhibit ligase activity and dialyzed for 2 h against ultrapure water through 0.025µm microporous membranes of 13 mm diameter (VSWP, Millipore). Ligation products were electroporated into the DH10B E. coli strain (Invitrogen), using the electroporator Easyject Plus (Eurogentec) with the following parameters: 2500 V, 25 µF, 201 W, 5 ms. Ligation products were plated on LB-agar supplemented with 100 µg/ml ampicillin in the presence of X-Gal (20 µg/ ml) and IPTG (200 µg/ml) for blue-white color selection of recombinant clones. Since the average insert size of the LBAB BAC library is 110 kb (Rogel-Gaillard et al. 2001), approximately 5000 white clones were plated for 20 BAC DNA pools. This number corresponds to a twofold coverage of each BAC with a plasmid sublibrary comprising 1 kb inserts and includes a 10% bacterial DNA contamination, as it is frequently observed in BAC DNA samples extracted with simple alkaline lysis procedures. Over 12 000 clones were plated for the genomic library. Clones were transferred onto Hybond+ nylon membranes (Amersham Pharmacia Biotec) and further treated for bacterial lysis, DNA denaturation, and neutralization (Sambrook et al. 1989). DNA was fixed on membranes by UV for 5 min. Membranes were hybridized with an oligonucleotide (TC) 12 and (TG) 12 probe labeled with the 3'DIG Labelling Kit (Boehringer Mannheim). Hybridizations were performed using the DIG Luminescent Detection Kit for Nucleic Acids in conditions recommended by the manufacturer (Boehringer Mannheim). A secondary screening of positive clones was performed in the same hybridization conditions.
All the positive clones were first sequenced on one strand with a direct universal primer (5'-GTTTTCCCAGTCACGAC-3', identified as SEQ ID N°8) and some of them were also sequenced on the second strand with a reverse universal primer (5'-CAGGAAA CAGCTATGACC-3', identified as SEQ ID N°9). DNA sequencing reactions were performed using Big Dye Terminator Kit (Applied Biosystems) and electrophoreses were run on an automatic sequencer ABI Prism 377A (Applied Biosystems). Vector and poor-quality sequences were removed. Microsatellite primers were designed in the tandem repeat flanking sequences using the Primer 3 program (Rozen and Skaletsky 2000).

An *in silico* approach based on comparative mapping was also used. The rabbit sequences available (777 141 sequences present in Ensembl (05/06/07), including Traces, EST, contigs, scaffolds and not located on the genome) were compared by BLAST with the human masked sequences of chromosome 3 for the 170Mb-190Mb interval of primary location (chromosome 14q17). Only sequences with a significant BLAST score, set with an "e" value greater than or equal to 10⁻⁴⁰ were kept. Microsatellite identification in these sequences has been implemented using TandemRepeatFinder (Benson, 1999). Finally, primers to amplify the microsatellite panel were designed by using Primer3 (Rozen and Skaletsky, 2000). Three amplification sizes were chosen to allow multiplexing: 100 ± 20 bp, 150 ± 20 bp and 200 ± 20 bp. We chose to select uninterrupted microsatellite sequences with at least 18 repetitions in order to improve the probability of detecting polymorphism.

### b. Mapping

The CRI-MAP software version 2.4 (Green et al., 1990) was used to construct the regional map. It allows the construction of linkage groups by the maximum likelihood method. We used MultiMap to generate maps and calculate the probabilities for orders obtained from an initial order of reference, by permutation of n adjacent loci.

### c. Linkage analysis

Taking into account the family structure, the number of individuals and the number of markers, we used Simwalk2 version 2.91 (Sobel et al., 2001) to perform linkage analysis based on the Z score method. A parametric test assuming a recessive model with complete penetrance and a nonparametric test, with the respective options "parametric linkage" analysis and "non-parametric linkage" (NPL) analysis have been made.

Haplotype analysis was also conducted with Simwalk2 v 2.91 and results were analysed using Haplopainter version 27beta (Thiele, 2006) and Gostscript 8.54.

### 4. Characterization of the rex gene

Exonic PCR amplifications of genes were performed for rex and Wild type rabbits. PCR amplifications were performed with 30 ng of gDNA, 1.5 mM of MgCl2, 0.5 mM of dNTP, 1 µM of each primer and 1.5 units of GoTaq® Flexi DNA Polymerase. The cycling conditions included an initial incubation at 94°C for 5 min followed by 35 cycles comprising 30 s at 94°C, 30 s of annealing in the range 56-62°C and 30 s at 72°C. The PCR products were sequenced directly and the exon-intron boundaries were established by comparing genomic and cDNA sequences. The primers used are presented in Table 2.

The sequences were assembled in clusters of contiguous sequences using the CAP3 assembler (Huang and Madan, 1999) using default parameters.

LIPH sequences for 2 rex and 2 wild type rabbits were compared using "multalign" (Corpet, 1988) and SNP detection was performed with the NovoSNP software (Weckx et al., 2005).

### 5. Association study

A panel of 60 wild type rabbits and 60 rex rabbits were genotyped by direct sequencing for the deletion using primers Ex_9F/ Ex_9R (see table 12.

**Table 2 : Primers used for cloning the rex gene**

| **Name** | **Sequence** | **SEQ ID N°** | **Amplicon size** |
|---|---|---|---|
| Ex 1_F | GCCAGGCACCATTCTAAAAG | 10 | 345 |
| Ex 1_R | TGAAAAGAGGAGGAGCCAAA | 11 | |
| Ex 2_F | TGGGGCTTATTCAGATTTGC | 12 | 574 |
| Ex 2_R | TGCAGTGAATAGCAGAGGATTC | 13 | |
| Ex 3_F | CAGCTAGGACACTTCTCCAAA | 14 | 292 |
| Ex 3_R | TGGAAAAGCTGGCTTTGAAC | 15 | |
| Ex 4_F | ACGAACCATCGAATCAGGAA | 16 | 267 |
| Ex 4 R | GCTACCCCCAGGGAGAGACT | 17 | |
| Ex 5_F | GCTCCCTCTCTCGCTGTAAC | 18 | 400 |
| Ex 5_R | CGCAGCTAACTATTGGGGTAA | 19 | |
| Ex 6_F | AGGGAAACTGCTTGTTGGAC | 20 | 208 |
| Ex 6_R | CTCTACAAAGCCAGGGATGC | 21 | |
| Ex 7_F | AGAAGTGGCTGGGAACCTG | 22 | 262 |
| Ex 7_R | CCAATGCGCTGTTCTCATTA | 23 | |
| Ex 8_F | AGAGCAGAAGTGCAGAACCA | 24 | 299 |
| Ex 8_R | GTTATCAGGGGGATGGGTTT | 25 | |
| Ex 9_F | TCTCCCTGACTTTTCCTACTTCA | 26 | 298 |
| Ex 9_R | CCCTCCCCAAATAAATCTTTTAAC | 27 | |
| Ex 10_F | ACACTGCAGAGAAGGCAGGT | 28 | 836 |
| Ex 10_R | AGCGTGGCTCCTGTTCATTA | 29 | |

### Conclusion

This mutation leads to a frameshift and a premature stop codon that results in a predicted truncated protein. The distribution of the 1362de1A allele among 60 rex and 60 inbred lines of rabbits was investigated. All the 60 rex rabbits were tested positive for the 1362de1A while all the 60 non rex rabbits were tested negative, as described in Figure 2.

### References

Chantry-Darmon C, Urien C, de Rochambeau H, Allain D, Pena B, Hayes H, Grohs C, Cribiu EP, Deretz-Picoulet S, Larzul C, Save JC, Neau A, Chardon P, Rogel-Gaillard C. A first-generation microsatellite-based integrated genetic and cytogenetic map for the European rabbit (Oryctolagus cuniculus) and localization of angora and albino. Anim Genet. 2006 Aug;37(4):335-41.
Chantry-Darmon C, Bertaud M, Urien C, Chadi-Taourit S, Perrocheau M, Rogel-Gaillard C, Hayes H. Expanded comparative mapping between man and rabbit and detection of a new conserved segment between HSA22 and OCU4. Cytogenet Genome Res. 2005;111(2):134-9.
Chantry-Darmon C, Urien C, Hayes H, Bertaud M, Chadi-Taourit S, Chardon P,Vaiman D, Rogel-Gaillard C. Construction of a cytogenetically anchored microsatellite map in rabbit. Mamm Genome. 2005 Jun;16(6):442-59.
Chantry-Darmon C, Rogel-Gaillard C, Bertaud M, Urien C, Perrocheau M, Chardon P, Hayes H. 133 new gene localizations on the rabbit cytogenetic map. Cytogenet Genome Res. 2003;103(1-2):192-201.
Chantry-Darmon C, Construction d'une carte intégrée génétique et cytogénétique chez le lapin européen (Oryctolagus cuniculus : application à la primo localisation du caractére rex. Thèse de Docteur de l'Université de Versailles Saint-Quentin, 2005.
Corpet F, Multiple sequence alignment with hierarchical clustering. 1988, Nucl. Acids Res., 16 (22), 10881-10890.
Green P (1988) Rapid construction of multilocus genetic linkage maps. I. Maximum likelihood estimation (draft manuscript).
Huang X, Madan A. CAP3: A DNA sequence assembly program. Genome Res. 1999 Sep;9(9):868-77.
Jeanpierre M. A rapid method for the purification of DNA from blood. Nucleic Acids Res. 1987 Nov 25;15(22):9611.
Létard E. Le lapin Castorrex. L'histoire d'une mutation. Rev. vét. et journ. de méd. vét. et de zoothechnie réunis. 1928. T80.
Lienhart R. Mise au point des connaissances biologiques actuelles sur le lapin Castorrex. Bulletin de la Société Lorraine des Sciences. 1962 ; 2 :84-102.
Rafat S.A., Rochambeau H. de, Brims M., Thebault R.G., Deretz S., Bonnet M. and Allain D., 2007. Characteristics of Angora rabbit fiber using optical fiber diameter analyzer, Journal of Animal Science, 85:3116-3122, doi:10.2527/jas.2007-0109.
Rogel-Gaillard C, Piumi F, Billault A, Bourgeaux N, Save JC, Urien C, Salmon J, Chardon P. Construction of a rabbit bacterial artificial chromosome (BAC) library: application to the mapping of the major histocompatibility complex to position 12q.1.1. Mamm Genome. 2001;12(3):253-5.
Rozen S et Skaletsky H J. Primer3 on the WWW for general users and for biologist programmers. In: Krawetz S, Misener S (eds) Bioinformatics Methods and Protocols: Methods in Molecular Biology. Humana Press, Totowa, NJ, 2000, pp 365-386.
Sambrook J, Gething MJ. Protein structure. Chaperones, paperones. Nature. 1989 Nov 16;342(6247):224-5.
Schuelke M. (2000) An economic method for the fluorescent labeling of PCR fragments. Nature Biotechnology 18, 233-4.
Sobel E, Sengul H, Weeks DE (2001) Multipoint estimation of identity-by-descent probabilities at arbitrary positions among marker loci on general pedigrees. Human Heredity 52:121-131.
Thiele H, Nürnberg P. HaploPainter: a tool for drawing pedigrees with complex haplotypes. Bioinformatics. 2005 Apr 15;21(8):1730-2. Epub 2004 Sep 17.
Weckx S, Del-Favero J, Rademakers R, Claes L, Cruts M, De Jonghe P, Van Broeckhoven C, De Rijk P. novoSNP, a novel computational tool for sequence variation discovery. Genome Res. 2005 Mar;15(3):436-42.
Wheelan SJ, Church DM, Ostell JM. Spidey: a tool for mRNA-to-genomic alignments. Genome Res. 2001 Nov;11(11):1952-7.
Sheils, O., Finn, S. and O'Leary J. (2003) "Nucleic acid microarray: an overview", Current Diagnostic Pathology. 9:155-158.
Barbas CF : Semisynthetic combinatorial antibody libraries: A chemical solution to the diversity problem. Proc. Nati. Acad. Sci. USA Vol. 89, pp. 4457-4461, May 1992.
Hannon and Rossi. Unlocking the potential of the human genome with RNA interference. Nature 2004 Sep 16; 431 (70006): 371-8.
Waterhouse et al: Combinatorial infection and in vivo recombination: a strategy for making large phage antibodies repertory. Nucl. Acids Res..1993; 21: 2265-2266.
Harlow et al. Antibodies: a laboratory manual, Cold Spring Harbor Laboratory Press, 1988.

## Claims

1. A method for identifying a fur-bearing animal having at least 95 % of down hair, wherein said method comprises the step of detecting the presence of a mutation in the lipase H (LIPH) gene in a biological sample obtained from said fur-bearing animal.

2. The method of claim 1, wherein said fur-bearing animal is a mammal selected in the group comprising sheep, goat, mouflon, camel, alpaca, llama, guanaco, vicuna, cow, horse, beaver, dog, cat, fox, mink, muskrat, nutria, weasel, polecat, and rabbit.

3. The method according to claim 1 wherein said fur bearing animal is a rabbit, preferably *Oryctolagus cuniculus*

4. The method of any one of claim 1 to 3, wherein said mutation in the lipase H gene is selected in the group comprising insertions, deletions, and point mutations corresponding to missense mutation and nonsense mutations.

5. The method of any one of claim 1 to 4, wherein said mutation in the lipase H gene is a non silent mutation.

6. The method of any one of claim 1 to 5, wherein said mutation in the lipase H gene is a deletion in the exon 9 of the lipase H gene.

7. The method of any one of claim 1 to 6, wherein said mutation in the lipage H gene is 1362delA in exon 9 of lipase H gene.

8. The method of any one of claim 1 to 7, wherein the step of detecting the presence of a mutation in the lipase H gene is realized on each allele of the LIPH gene of said fur bearing animal.

9. The method of any one of claim 1 to 8, wherein the fur-bearing animal have at least 95% of down hair, preferably at least 98% of down hair, more preferably 100% of down hair.

10. The method of any one of claim 1 to 9, wherein the fur-bearing animal has the REX phenotype.

11. The method of any one of claim 1 to 10, wherein said method further comprises a step (i'), before step (i), of transformation of cells of said animal for presenting a mutation in the lipase H gene and/or under-expressing the lipase H gene or expressing a truncated lipase H protein.

12. Non-human transgenic fur-bearing animal obtained by the method according to claim 11.

13. Non-human transgenic fur-bearing animal according to claim 12 wherein said animal is a rabbit.

14. Use of a compound that specifically inhibit the expression of lipase H gene for obtaining fur-bearing animals having at least 95% of down hair.

15. Use according to claim 14, wherein said compound is an oligonucleotide, selected in the group comprising anti-sens DNA and RNA molecules, ribozymes, SiRNA and aptamers.


SEQUENCE LISTING

<110>  INRA

<120>  Obtention of a rex animal by molecular methods based on the
       alteration of the LIPH function

<130>  BEP090541

<160>  29

<170>  PatentIn version 3.3

<210>  1
<211>  1978
<212>  DNA
<213>  Oryctolagus cuniculus

<400>  1
agttatctga gctcgccgag agcagaacca ggggaaacct aaagctccgc gcgaaggaat      60

cgttccagcg actgtgacaa gtattctctc cagtttccat atgctgagat tttatttgtt     120

catcagtttg ctgtgcttgg tgagatcaga cacagacgaa acatgtcctt cattcaccaa     180

actgagcttt cacagtgcag tggttggtac ggaactcaat gtgaggttgt tgctctacac     240

caggaaaaac tacacctgcg cacaaatcat caactccaca acttttggaa acttgaatgt     300

gaccaagaaa accaccttcg ttgtccatgg attcagacca acaggctccc ctccagtttg     360

gttgcaggac ttagtaaaag ctttgctcat ggtagaagac atgaacctgg ttgttgttga     420

ttggaatcgt ggagctacaa ctgtaatata cacccaggcc tctaataaga ccaggaaagt     480

agccataatc ttgaaggagt ttatcgacca gatgttggca agaggagcct ctctggatga     540

catctacatg attggagtaa gtctaggagc ccacatatca gggtttgttg gaaagatgta     600

caatgggcag ctggggagaa ttacaggtct cgaccctgcg ggccctttat tcaacgggaa     660

gcctccccag gacagattag atcccagtga tgcgcagttc gttgatgtca tccattccga     720

cactgacgca ctgggctata aggaaccatt aggaaacata gacttctacc caaatggagg     780

agtggatcaa cctggttgcc ccaagacaat ctttgaagca gggatgcagt actttaaatg     840

tgaccaccag atgtccgtgt acctgtacct ttcctccctg agaaagaact gtaccatcac     900

tgcatacccc tgcgactcct acagggatta caggaatggc aagtgcatca actgcggcct     960

accccaaggg aagccctgcc ccctgctggg ctactatgct gacaactgga aagattattt    1020

aagtgagaaa gaccctccaa tgactaaagc attctttgat acagctgaga aggagccata    1080

ctgcatgtat cattattttg tggatattat aacttggaac aagagcatca gaagagggtc    1140

cattacaatc aaattgaaag atgaagctgg aaacaccaca gaatccaaaa tcaatcatga    1200

accagtcaca tttgagaaat atcatcaagt gagtctgctt gcaagattta atcaagatct    1260

ggataaagtg gcagaaattt ccttggtgtt ctccacggga gctgtaatag gcccaaagta    1320

caagctcagg attctccgaa tgaagttaag gtcgcttgcc catccagaga ggccccagtt    1380

gtgtcggtat gatctcgtcc tgacggaaaa tgtggaaaca cccttccaac ccattgtttg    1440

ccagaagctg cagatgtagc tgttgttggg acacacacag accagggaca tcgagcaggc    1500

tgacactttc tgagcttcct ctttactttt cctcaaggca caaaaagaac tgaaaaactg 1560
aggatgtcac attataaaac atcaaacgac cttgtggtta taaaaccatc ctgggaaggg 1620
agcccctaac tagggcaagt cagaaatagc caggctctgc cagcccagcg ctttgtctcg 1680
ctgcatcctg gggcctcatt tgttccgagc tgtcgattct gctgtccatc agcacagcga 1740
ttctgcccat catgctgcgg gtcaagcatc ttcaagggaa agacgggctg gaggtctccc 1800
tgtagactgg gctccacatc ttctgtgcca gtccttccag ttccccactg gtagaagggg 1860
atgaaactga cctacctcac agggctgttg tgggggaggc aaattgatga aggggttttg 1920
aaaatctcat gggtgtcaca tctgtgagat gtttgataaa tgtgaattat ttactttg 1978

<210> 2
<211> 452
<212> PRT
<213> Oryctolagus cuniculus

<400> 2

Met Leu Arg Phe Tyr Leu Phe Ile Ser Leu Leu Cys Leu Val Arg Ser
1               5                   10                  15

Asp Thr Asp Glu Thr Cys Pro Ser Phe Thr Lys Leu Ser Phe His Ser
            20                  25                  30

Ala Val Val Gly Thr Glu Leu Asn Val Arg Leu Leu Leu Tyr Thr Arg
        35                  40                  45

Lys Asn Tyr Thr Cys Ala Gln Ile Ile Asn Ser Thr Thr Phe Gly Asn
    50                  55                  60

Leu Asn Val Thr Lys Lys Thr Thr Phe Val Val His Gly Phe Arg Pro
65                  70                  75                  80

Thr Gly Ser Pro Pro Val Trp Leu Gln Asp Leu Val Lys Ala Leu Leu
                85                  90                  95

Met Val Glu Asp Met Asn Leu Val Val Val Asp Trp Asn Arg Gly Ala
            100                 105                 110

Thr Thr Val Ile Tyr Thr Gln Ala Ser Asn Lys Thr Arg Lys Val Ala
        115                 120                 125

Ile Ile Leu Lys Glu Phe Ile Asp Gln Met Leu Ala Arg Gly Ala Ser
    130                 135                 140

Leu Asp Asp Ile Tyr Met Ile Gly Val Ser Leu Gly Ala His Ile Ser
145                 150                 155                 160

Gly Phe Val Gly Lys Met Tyr Asn Gly Gln Leu Gly Arg Ile Thr Gly
                165                 170                 175

Leu Asp Pro Ala Gly Pro Leu Phe Asn Gly Lys Pro Pro Gln Asp Arg

            180                 185                 190

Leu Asp Pro Ser Asp Ala Gln Phe Val Asp Val Ile His Ser Asp Thr
        195                 200                 205

Asp Ala Leu Gly Tyr Lys Glu Pro Leu Gly Asn Ile Asp Phe Tyr Pro
    210                 215                 220

Asn Gly Gly Val Asp Gln Pro Gly Cys Pro Lys Thr Ile Phe Glu Ala
225                 230                 235                 240

Gly Met Gln Tyr Phe Lys Cys Asp His Gln Met Ser Val Tyr Leu Tyr
                245                 250                 255

Leu Ser Ser Leu Arg Lys Asn Cys Thr Ile Thr Ala Tyr Pro Cys Asp
            260                 265                 270

Ser Tyr Arg Asp Tyr Arg Asn Gly Lys Cys Ile Asn Cys Gly Leu Pro
        275                 280                 285

Gln Gly Lys Pro Cys Pro Leu Leu Gly Tyr Tyr Ala Asp Asn Trp Lys
    290                 295                 300

Asp Tyr Leu Ser Glu Lys Asp Pro Pro Met Thr Lys Ala Phe Phe Asp
305                 310                 315                 320

Thr Ala Glu Lys Glu Pro Tyr Cys Met Tyr His Tyr Phe Val Asp Ile
                325                 330                 335

Ile Thr Trp Asn Lys Ser Ile Arg Arg Gly Ser Ile Thr Ile Lys Leu
            340                 345                 350

Lys Asp Glu Ala Gly Asn Thr Thr Glu Ser Lys Ile Asn His Glu Pro
        355                 360                 365

Val Thr Phe Glu Lys Tyr His Gln Val Ser Leu Leu Ala Arg Phe Asn
    370                 375                 380

Gln Asp Leu Asp Lys Val Ala Glu Ile Ser Leu Val Phe Ser Thr Gly
385                 390                 395                 400

Ala Val Ile Gly Pro Lys Tyr Lys Leu Arg Ile Leu Arg Met Lys Leu
                405                 410                 415

Arg Ser Leu Ala His Pro Glu Arg Pro Gln Leu Cys Arg Tyr Asp Leu
            420                 425                 430

Val Leu Thr Glu Asn Val Glu Thr Pro Phe Gln Pro Ile Val Cys Gln
        435                 440                 445

Lys Leu Gln Met
    450

<210> 3
<211> 3811
<212> DNA
<213> Mus musculus

<400> 3
gacaggacta acagggccac agagcctccc tcggggactt cctgttgtca cggactcact 60

cgcttcgaga aagccagact tcagggtggg acgcgggaac gcgggaacgc caggctctgt 120

gcggagtgcg acacgtcctc gtgtttgggg cacatgatct cttgaggttc cctatgctga 180

ggctatgttt tttcatcagt ttcatgtgct tggtgaaatc agacacggat gaaacatgcc 240

cgtccttcac cagactgagc ttccacagcg cggtggttgg tacaggactc tccgtgagac 300

tgatgctcta cacacagaga gaccagacct gtgcacagat catcaactcc acagctctgg 360

ggagcttaaa tgtgaccaag aaaaccactt ttataatcca cggtttccgg ccaacaggct 420

cccctccggt ttggatagag gagctggtac agagtttgat cagtgtacaa gagatgaatg 480

tagtggttgt tgattggaat cgaggagcga caactgtgat atacccccat gcgtctagca 540

agaccagaca agtagcttct attttgaagg aatttattga ccagatgttg gtcaaaggag 600

cttctctgga caacatttac atgattggag taagtctagg agcccacata gctggatttg 660

ttggcgagtc gtatgagggg aagctgggga gagtcacagg tcttgaccct gcaggccctt 720

tattcaacgg gagacctcct gaggagagat tagaccccag cgacgcactg ttcgtggacg 780

tcatccactc tgacactgac gcactaggct acaaggaagc cctaggacac atagacttct 840

acccgaatgg aggactagat caacccggtt gccccaagac aatatttgga ggtataaagt 900

acttcaagtg tgaccaccag atgtccgtct acctgtacct tgcatccctg caaaataact 960

gctccatcac ggcctatccc tgcgactcct atcgggatta taggaatggc aagtgtgtca 1020

gctgtggcgc tggacaaatt gtgccatgtc cccgtgtagg ctactatgct gacagctgga 1080

aagagtactt atgggacaga gatcctccga tgacgaaggc gttcttcgac acagctgaga 1140

caaagccata ctgcatgtat cattattttg tggacattgt atcttggaac aagagcgtaa 1200

gaagagggtt tattacaatc aaactgagag gcgaagatgg caatatcaca gaatccaaaa 1260

tcgatcatga gccatctgca tttgagaaat accatcaagt gagtctcctc gcaagattta 1320

atcgagatct ggataaggtg gcagagattt ccttgttgtt ctccacaggg tctgtagtgg 1380

gcccaaagta caagctcagg gtcctgcaga tgaagctgag gtctctggcc catccagaca 1440

ggcctcactt gtgtcgatat gatctcgtcc ttatggagaa tgttgagaca tccttccaac 1500

ctatcctgtg ctctcagcag cagatgtagc tgctgttaag acctgtgggc agtgatgaca 1560

tcaagatctc tacagctata ggctttcagt gcctcaccct atagcgtcct gaaggcttcc 1620

aaagcatggg gaagaaaacc aaggttattt tcggctgtgt tccaaggatg tcgcgtcaca 1680

aaaatgtcaa aggacctgcg attatgaaac accactggga gggggggggc gccctaacta 1740

gggcaagaca gaaatagccc agctccctac agcaaagcac tttgtctggc tgcgtcccga 1800

ggccccactt gttccccact gtcgaatctg ctgcctgtca taccacaccc tcagggccgt 1860

ggggcaagca tctaaatggt ccctgtgccc aatcctctgc catctcggcg ggaaccgaat 1920
ggagaatgaa ccagactacc tcacagggac tgtgggtggg gagggccagg ctgtgaaggg 1980
tttggaaatc ccttgggtcc cttgggtgga ctactgtgaa catgcttttc ttttgatagg 2040
gtttggctct tacgtaaaga cacacaatct ctctttttca acaccagctt tcctcctgga 2100
ctgtttgcct cggggcgaca agaaggctct ggcttagtcc agactcttct gaagtgatgc 2160
tgatggccag aggtattggg ctgggctcat agccctgagg cctgtgcagc taagtggaag 2220
aaagagaaaa aacaaaaaaa aaacaaaaac aaacagggct agcattgcag catgtggact 2280
gggcagagaa aaggctggaa ggaaactggc ctgctttcac tcaagttgcg aaagtgttaa 2340
ttacaaggtt gtttttctgc tctgggattg agtatgctaa gtgcactgag ccctgctctc 2400
agcttcagac attttttaaa agtagtcctt aagactgttt tcttaaaaaa caaaaacaaa 2460
agctgggcag tggtggagca caactttaac cccagcactt gagaggagag gcaggcagat 2520
ctctgtgagt ttgaggacag cctggtctat agagcaagtt ccgggacaac cagggctaca 2580
cggagaaacc ctgtcttcaa aacaaaaaca aacaaatgaa agttttcttt ccttcctatt 2640
ggtgcaatcc atacttattc caggaaattc cttaagatgt atagatgtaa aatacaaaac 2700
tgtgcacagt cctgcctgtt agaggaatta atgttaatat ttagacttgt ttctcaaact 2760
tgtggggctc agattcagag catggccggc tcttggggca tggaaactct gagaagtctt 2820
cccaggcctc cctggaggca gatgtgactt caaacatttc tgaataacag tggcaaacag 2880
attaaggggt cacagggggc cagggacaga gggcaaaatg aaagtagaac aggtagaggg 2940
tccagacgcc tggtgtggag gtgagtgccc cccacccctc atctgtgacc actgggggaa 3000
ggtgtcacag gcccagggcc cttctcttca ttgaattgcc ccagctattg taaagaaatg 3060
gatactgtga gcaaatgtgt tgaaatccac actgtcttag tttcttttcc cgttgctatg 3120
ataaaatgct ctgatgaaaa caacgtgaag gagaaagggt ttattttagc tcgcagttca 3180
gggtatacag tccactgtga cagggagtcg gggtagcagc atcgtaaaag cagctggtcc 3240
tatcgctgcc acagtcagga agcagggcga tgaacacatg cttgcgagta tttagctcag 3300
tttcctgaca catttaaaaa ttgttaaact ttgttaattt atagcgtgtg tttgtatttg 3360
tacacacatg tacacatgtc acacacagtg cacatgtgga gttcagaagg aaatttgtgg 3420
gagtcagttc tctccttcca atgtgtgggt cccaggtatc tgggcattag gcttagaggc 3480
aagtatgttt accacctgag ccatctcact ggactagttt cccagctctt acacaattca 3540
gattccctga ctagggaatg gtgccaccta caatgggcag gtgtccataa atataatcaa 3600
gatcatttct cgggctgtac agaaggctca gtggttaaga gcactgactg ttcttccaga 3660
ggtcctgagt tcaatatcca acaactacct ggtggctcac aaccatctgt aataggatct 3720
gatgctctct tctggtgtgt gtgaagacag ctacagtgga ctcataaata taaaaataaa 3780
taaataagtc tttaaaaaga aaaaaaaaaa a 3811

<210> 4

<211>  451
<212>  PRT
<213>  Mus musculus

<400>  4

Met Leu Arg Leu Cys Phe Phe Ile Ser Phe Met Cys Leu Val Lys Ser
1               5                   10                  15

Asp Thr Asp Glu Thr Cys Pro Ser Phe Thr Arg Leu Ser Phe His Ser
            20                  25                  30

Ala Val Val Gly Thr Gly Leu Ser Val Arg Leu Met Leu Tyr Thr Gln
        35                  40                  45

Arg Asp Gln Thr Cys Ala Gln Ile Ile Asn Ser Thr Ala Leu Gly Ser
    50                  55                  60

Leu Asn Val Thr Lys Lys Thr Thr Phe Ile Ile His Gly Phe Arg Pro
65                  70                  75                  80

Thr Gly Ser Pro Pro Val Trp Ile Glu Glu Leu Val Gln Ser Leu Ile
                85                  90                  95

Ser Val Gln Glu Met Asn Val Val Val Val Asp Trp Asn Arg Gly Ala
            100                 105                 110

Thr Thr Val Ile Tyr Pro His Ala Ser Ser Lys Thr Arg Gln Val Ala
        115                 120                 125

Ser Ile Leu Lys Glu Phe Ile Asp Gln Met Leu Val Lys Gly Ala Ser
    130                 135                 140

Leu Asp Asn Ile Tyr Met Ile Gly Val Ser Leu Gly Ala His Ile Ala
145                 150                 155                 160

Gly Phe Val Gly Glu Ser Tyr Glu Gly Lys Leu Gly Arg Val Thr Gly
                165                 170                 175

Leu Asp Pro Ala Gly Pro Leu Phe Asn Gly Arg Pro Pro Glu Glu Arg
            180                 185                 190

Leu Asp Pro Ser Asp Ala Leu Phe Val Asp Val Ile His Ser Asp Thr
        195                 200                 205

Asp Ala Leu Gly Tyr Lys Glu Ala Leu Gly His Ile Asp Phe Tyr Pro
    210                 215                 220

Asn Gly Gly Leu Asp Gln Pro Gly Cys Pro Lys Thr Ile Phe Gly Gly
225                 230                 235                 240

Ile Lys Tyr Phe Lys Cys Asp His Gln Met Ser Val Tyr Leu Tyr Leu
                245                 250                 255

Ala Ser Leu Gln Asn Asn Cys Ser Ile Thr Ala Tyr Pro Cys Asp Ser
            260                 265                 270

Tyr Arg Asp Tyr Arg Asn Gly Lys Cys Val Ser Cys Gly Ala Gly Gln
        275                 280                 285

Ile Val Pro Cys Pro Arg Val Gly Tyr Tyr Ala Asp Ser Trp Lys Glu
    290                 295                 300

Tyr Leu Trp Asp Arg Asp Pro Pro Met Thr Lys Ala Phe Phe Asp Thr
305                 310                 315                 320

Ala Glu Thr Lys Pro Tyr Cys Met Tyr His Tyr Phe Val Asp Ile Val
                325                 330                 335

Ser Trp Asn Lys Ser Val Arg Arg Gly Phe Ile Thr Ile Lys Leu Arg
            340                 345                 350

Gly Glu Asp Gly Asn Ile Thr Glu Ser Lys Ile Asp His Glu Pro Ser
        355                 360                 365

Ala Phe Glu Lys Tyr His Gln Val Ser Leu Leu Ala Arg Phe Asn Arg
    370                 375                 380

Asp Leu Asp Lys Val Ala Glu Ile Ser Leu Leu Phe Ser Thr Gly Ser
385                 390                 395                 400

Val Val Gly Pro Lys Tyr Lys Leu Arg Val Leu Gln Met Lys Leu Arg
                405                 410                 415

Ser Leu Ala His Pro Asp Arg Pro His Leu Cys Arg Tyr Asp Leu Val
            420                 425                 430

Leu Met Glu Asn Val Glu Thr Ser Phe Gln Pro Ile Leu Cys Ser Gln
        435                 440                 445

Gln Gln Met
    450

<210>  5
<211>  2050
<212>  DNA
<213>  Mus musculus

<400>  5
gcgggaacgc gggaacgcca ggctctgtgc ggagtgcgac acgtcctcgt gtttggggca      60

catgatctct tgaggttccc tatgctgagg ctatgttttt tcatcagttt catgtgcttg     120

gtgaaatcag acacggatga aacatgcccg tccttcacca gactgagctt ccacagcgcg     180

gtggttggta caggactctc cgtgagactg atgctctaca cacagagaga ccagacctgt     240

gcacagatca tcaactccac agctctgggg agcttaaatg tgaccaagaa aaccactttt     300

ataatccacg gtttccggcc aacaggctcc cctccggttt ggatagagga gctggtacag 360

agtttgatca gtgtacaaga gatgaatgta gtggttgttg attggaatcg aggagcgaca 420

actgtgatat acccccatgc gtctagcaag accagacaag tagcttctat tttgaaggaa 480

tttattgacc agatgttggt caaaggagct tctctggaca acatttacat gattggagta 540

agtctaggag cccacatagc tggatttgtt ggcgagtcgt atgaggggaa gctggggaga 600

gtcacaggtc ttgaccctgc aggcccttta ttcaacggga gacctcctga ggagagatta 660

gaccccagcg acgcactgtt cgtggacgtc atccactctg acactgacgg tataaagtac 720

ttcaagtgtg accaccagat gtccgtctac ctgtaccttg catccctgca aaataactgc 780

tccatcacgg cctatccctg cgactcctat cgggattata ggaatggcaa gtgtgtcagc 840

tgtggcgctg gacaaattgt gccatgtccc cgtgtaggct actatgctga cagctggaaa 900

gagtacttat gggacagaga tcctccgatg acgaaggcgt tcttcgacac agctgagaca 960

aagccatact gcatgtatca ttattttgtg gacattgtat cttggaacaa gagcgtaaga 1020

agagggttta ttacaatcaa actgagaggc gaagatggca atatcacaga atccaaaatc 1080

gatcatgagc catctgcatt tgagaaatac catcaagtga gtctcctcgc aagatttaat 1140

cgagatctgg ataaggtggc agagatttcc ttgttgttct ccacagggtc tgtagtgggc 1200

ccaaagtaca agctcagggt cctgcagatg aagctgaggt ctctggccca tccagacagg 1260

cctcacttgt gtcgatatga tctcgtcctt atggagaatg ttgagacatc cttccaacct 1320

atcctgtgct ctcagcagca gatgtagctg ctgttaagac ctgtgggcag tgatgacatc 1380

aagatctcta cagctatagg ctttcagtgc ctcaccctat agcgtcctga aggcttccaa 1440

agcatgggga agaaaaccaa ggttattttc ggctgtgttc caaggatgtc gcgtcacaaa 1500

aatgtcaaag gacctgcgat tatgaaacac cactgggagg gggggggcgc cctaactagg 1560

gcaagacaga aatagcccag ctccctacag caaagcactt tgtctggctg cgtcccgagg 1620

ccccacttgt tccccactgt cgaatctgct gcctgtcata ccacaccctc agggccgtgg 1680

ggcaagcatc taaatggtcc ctgtgcccaa tcctctgcca tctcggcggg aaccgaatgg 1740

agaatgaacc agactacctc acagggactg tgggtgggga gggccaggct gtgaagggtt 1800

tggaaatccc ttgggtccct tgggtggact actgtgaaca tgcttttctt ttgatagggt 1860

ttggctctta cgtaaagaca cacaatctct ctttttcaac accagctttc ctcctggact 1920

gtttgcctcg ggcgacaaag aaggctctgg cttagtccag actcttctga agtgatgctg 1980

atggccagag gtattgggct gggctcatag ccctgaggcc tgtgcagcta agtggaagaa 2040

agagaaaaaa 2050

<210> 6
<211> 421
<212> PRT
<213> Mus musculus

<400> 6

Met Leu Arg Leu Cys Phe Phe Ile Ser Phe Met Cys Leu Val Lys Ser

1               5                   10                  15

Asp Thr Asp Glu Thr Cys Pro Ser Phe Thr Arg Leu Ser Phe His Ser
            20                  25                  30

Ala Val Val Gly Thr Gly Leu Ser Val Arg Leu Met Leu Tyr Thr Gln
        35                  40                  45

Arg Asp Gln Thr Cys Ala Gln Ile Ile Asn Ser Thr Ala Leu Gly Ser
    50                  55                  60

Leu Asn Val Thr Lys Lys Thr Thr Phe Ile Ile His Gly Phe Arg Pro
65                  70                  75                  80

Thr Gly Ser Pro Pro Val Trp Ile Glu Glu Leu Val Gln Ser Leu Ile
                85                  90                  95

Ser Val Gln Glu Met Asn Val Val Val Val Asp Trp Asn Arg Gly Ala
            100                 105                 110

Thr Thr Val Ile Tyr Pro His Ala Ser Ser Lys Thr Arg Gln Val Ala
        115                 120                 125

Ser Ile Leu Lys Glu Phe Ile Asp Gln Met Leu Val Lys Gly Ala Ser
    130                 135                 140

Leu Asp Asn Ile Tyr Met Ile Gly Val Ser Leu Gly Ala His Ile Ala
145                 150                 155                 160

Gly Phe Val Gly Glu Ser Tyr Glu Gly Lys Leu Gly Arg Val Thr Gly
                165                 170                 175

Leu Asp Pro Ala Gly Pro Leu Phe Asn Gly Arg Pro Pro Glu Glu Arg
            180                 185                 190

Leu Asp Pro Ser Asp Ala Leu Phe Val Asp Val Ile His Ser Asp Thr
        195                 200                 205

Asp Gly Ile Lys Tyr Phe Lys Cys Asp His Gln Met Ser Val Tyr Leu
    210                 215                 220

Tyr Leu Ala Ser Leu Gln Asn Asn Cys Ser Ile Thr Ala Tyr Pro Cys
225                 230                 235                 240

Asp Ser Tyr Arg Asp Tyr Arg Asn Gly Lys Cys Val Ser Cys Gly Ala
                245                 250                 255

Gly Gln Ile Val Pro Cys Pro Arg Val Gly Tyr Tyr Ala Asp Ser Trp
            260                 265                 270

Lys Glu Tyr Leu Trp Asp Arg Asp Pro Pro Met Thr Lys Ala Phe Phe
        275                 280                 285

Asp Thr Ala Glu Thr Lys Pro Tyr Cys Met Tyr His Tyr Phe Val Asp
    290                 295                 300

Ile Val Ser Trp Asn Lys Ser Val Arg Arg Gly Phe Ile Thr Ile Lys
305                 310                 315                 320

Leu Arg Gly Glu Asp Gly Asn Ile Thr Glu Ser Lys Ile Asp His Glu
                325                 330                 335

Pro Ser Ala Phe Glu Lys Tyr His Gln Val Ser Leu Leu Ala Arg Phe
            340                 345                 350

Asn Arg Asp Leu Asp Lys Val Ala Glu Ile Ser Leu Leu Phe Ser Thr
        355                 360                 365

Gly Ser Val Val Gly Pro Lys Tyr Lys Leu Arg Val Leu Gln Met Lys
    370                 375                 380

Leu Arg Ser Leu Ala His Pro Asp Arg Pro His Leu Cys Arg Tyr Asp
385                 390                 395                 400

Leu Val Leu Met Glu Asn Val Glu Thr Ser Phe Gln Pro Ile Leu Cys
                405                 410                 415

Ser Gln Gln Gln Met
            420

<210>  7
<211>  17
<212>  DNA
<213>  Artificial

<220>
<223>  Primer

<400>  7
gaccggcagc aaaattg                                                    17

<210>  8
<211>  17
<212>  DNA
<213>  Artificial

<220>
<223>  primer

<400>  8
gttttcccag tcacgac                                                    17

<210>  9
<211>  18
<212>  DNA
<213>  Artificial

<220>
<223>  Primer

<400>  9
caggaaacag ctatgacc                                                    18


<210>  10
<211>  20
<212>  DNA
<213>  Artificial

<220>
<223>  Primer

<400>  10
gccaggcacc attctaaaag                                                  20


<210>  11
<211>  20
<212>  DNA
<213>  Artificial

<220>
<223>  Primer

<400>  11
tgaaaagagg aggagccaaa                                                  20


<210>  12
<211>  20
<212>  DNA
<213>  Artificial

<220>
<223>  Primer

<400>  12
tggggcttat tcagatttgc                                                  20


<210>  13
<211>  22
<212>  DNA
<213>  Artificial

<220>
<223>  Primer

<400>  13
tgcagtgaat agcagaggat tc                                               22


<210>  14
<211>  21
<212>  DNA
<213>  Artificial

<220>
<223>  Primer

<400>  14
cagctaggac acttctccaa a                                                21


<210>  15
<211>  20
<212>  DNA
<213>  Artificial

<220>
<223>  Primer

<400>  15
tggaaaagct ggctttgaac                                                    20


<210>  16
<211>  20
<212>  DNA
<213>  Artificial

<220>
<223>  Primer

<400>  16
acgaaccatc gaatcaggaa                                                    20


<210>  17
<211>  20
<212>  DNA
<213>  Artificial

<220>
<223>  Primer

<400>  17
gctaccccca gggagagact                                                    20


<210>  18
<211>  20
<212>  DNA
<213>  Artificial

<220>
<223>  Primer

<400>  18
gctccctctc tcgctgtaac                                                    20


<210>  19
<211>  21
<212>  DNA
<213>  Artificial

<220>
<223>  Primer

<400>  19
cgcagctaac tattggggta a                                                  21


<210>  20
<211>  20
<212>  DNA
<213>  Artificial

<220>
<223>  Primer

<400>  20
agggaaactg cttgttggac                                                    20


<210>  21

<211>  20
<212>  DNA
<213>  Artificial

<220>
<223>  Primer

<400>  21
ctctacaaag ccagggatgc                                                    20


<210>  22
<211>  19
<212>  DNA
<213>  Artificial

<220>
<223>  Primer

<400>  22
agaagtggct gggaacctg                                                     19


<210>  23
<211>  20
<212>  DNA
<213>  Artificial

<220>
<223>  Primer

<400>  23
ccaatgcgct gttctcatta                                                    20


<210>  24
<211>  20
<212>  DNA
<213>  Artificial

<220>
<223>  Primer

<400>  24
agagcagaag tgcagaacca                                                    20


<210>  25
<211>  20
<212>  DNA
<213>  Artificial

<220>
<223>  Primer

<400>  25
gttatcaggg ggatgggttt                                                    20


<210>  26
<211>  23
<212>  DNA
<213>  Artificial

<220>
<223>  primer

<400>  26
tctccctgac ttttcctact tca                                                23

<210>  27
<211>  24
<212>  DNA
<213>  Artificial

<220>
<223>  Primer

<400>  27
ccctccccaa ataaatcttt taac                                              24


<210>  28
<211>  20
<212>  DNA
<213>  Artificial

<220>
<223>  Primer

<400>  28
acactgcaga gaaggcaggt                                                   20


<210>  29
<211>  20
<212>  DNA
<213>  Artificial

<220>
<223>  Primer

<400>  29
agcgtggctc ctgttcatta                                                   20